# EUROPEAN PATENT SPECIFICATION

(11) **EP 3 833 316 B1**
(45) Date of publication and mention of the grant of the patent: **02.11.2022**
(21) Application number: 19753323.5
(22) Date of filing: 09.08.2019
(51) Int. Cl.: A61K 8/02, A61K 8/11, A61K 8/34, A61K 8/81, A61K 8/86, A61Q 5/06

(54) **LIQUID CRYSTAL HAIR DYE COMPOSITION, USE AND METHOD**
FLÜSSIGKRISTALLHAARFÄRBEZUSAMMENSETZUNG, VERWENDUNG UND VERFAHREN
COMPOSITION DE COLORANT CAPILLAIRE À CRISTAUX LIQUIDES, UTILISATION ET PROCÉDÉ

(30) Priority: 10.08.2018 GB 201813099
(43) Date of publication of application: 16.06.2021
(73) Proprietor: Theunseen Limited, London N1 7UX (GB)
(72) Inventor: BOWKER, Lauren, London N1 7UX (GB); ANDERSON, Louise, London N1 7UX (GB); PASHTOON, Palwasha, London N1 7UX (GB); GOULDING, David, London N1 7UX (GB)
(74) Representative: Geary, Stephen
(86) International application number: PCT/EP2019/071513
(87) International publication number: WO 2020/030818

(56) References cited:
- FR-A1- 2 705 230
- FR-A1- 2 750 601
- JP-A- S61 281 171
- JP-A- 2001 122 741
- US-A1- 2003 129 247
- Anonymous: "Handbook of Thermochromic Liquid Crystal Technology", , 8 September 2015 (2015-09-08), XP055637115, Retrieved from the Internet: URL:https://web.archive.org/web/2015090811 2119if_/http://www.hallcrest.com:80/Deskto pModules/Bring2mind/DMX/Download.aspx?Comm and=Core_Download&EntryId=280&language=en- US&PortalId=0&TabId=163 [retrieved on 2019-10-29]

## Description

This invention relates to a liquid crystal composition, use of the composition in colouring a substrate and to a method of colouring a substrate. The invention is particularly concerned with a composition comprising an encapsulated liquid crystal for colouring a keratinous material, especially hair.

Treating substrates to provide colour has been known since the earliest times using natural pigments and dyes. In modern times, synthetic dyes, pigments, leuco dyes and liquid crystals have been used to provide colour to a substrate. Leuco dyes and liquid crystals may also provide a colour-change effect dependent on an outside stimulus such as light (photochromic) or heat (thermochromic).

Liquid crystals comprise optically active organic chemicals which may be sealed, for example by microencapsulation, or unsealed for example in a poly-disperse form, as an oil. Liquid crystals may be encapsulated through a variety of means and materials, for example via complex coacervation with gum arabic and gelatin.

Liquid crystals may provide a constant or fixed colour, referred to herein as a "static" colour or, with a change in external stimulus such as temperature, may provide a change of colour from clear to coloured or vice-versa or between a first and a second colour. Typically, liquid crystals are either "cholesteric" or "chiral nematic" liquid crystals and may be employed alone or in mixtures with each other and with other colour materials. Cholesteric liquid crystals are typically based on or comprise cholesterol or other sterol derived compounds. Chiral nematic liquid crystals typically comprise non-sterol based compounds.

Liquid crystals show colour by selectively reflecting incident white light. When deposited as a thin film, liquid crystals may reflect particular wavelengths of light according to the temperature, for example as the temperature is increased, the liquid crystal may change from colourless to red through the other colours of the visible spectrum in sequence and then colourless again at a higher temperature, known as the "clearing point". When the liquid crystal is in its colourless state, the underlying colour of the substrate or other components in the composition will be visible. Such colour changes are reversible such that on cooling the colour change occurs in reverse. The temperature at which commencement of colour change, referred to as the "starting point" or "clearing point" occurs and the temperature spread over which colour change occurs, referred to as the "bandwidth" may be tuned according to the composition of the liquid crystal composition. Colour materials may be applied to a hair fibre as a coating to provide a temporary colour effect or through chemical interaction by penetrating the hair cuticle. Hair fibres typically have a diameter of 15 to 150 microns. Colour materials such as, pigments, synthetic dyes typically have a much smaller particle size than hair fibres, allowing the dye or pigment to penetrate the hair cuticle. Materials such as hydrogen peroxide or ammonia are often employed in dyeing hair to cause the hair fibre to swell thereby aiding penetration of the dye or pigment for permanent bonding to the fibre, providing a semi-permanent or permanent effect.

Encapsulated liquid crystals are typically much larger than dyes, leuco-dyes and pigments, typically having a particle size from 5 to 50 microns, for example from 10 to 20 microns, a similar order of magnitude to hair fibres. This presents some challenges in seeking to adhere liquid crystals to hair fibres. Due to the relatively similar size of encapsulated liquid crystals and the hair fibre, a relatively thick coating is required to accommodate the encapsulate. In addition, a vibrancy of colour is aesthetically desirable but encapsulated liquid crystals may require high loadings of encapsulate to achieve a suitable intensity or vibrancy of colour. Known hair dyeing techniques typically require a pre-treatment to lighten hair in order to obtain brighter dyed hair, for example green dye when applied to dark hair does not typically provide an acceptable colouration unless the hair is pre-lightened. However, higher loadings with attendant poor "feel" qualities and prior lightening treatments are undesirable.

JP2001122741 A discloses cosmetics which comprise a cholesteric photochromic or thermochromic liquid crystal compound that imparts hair with specific color effects. The cosmetics may comprise polyols.

We have now found that a surface colour coating of an encapsulated liquid crystal to provide a temporary hair colour effect with vibrant colour may be achieved without a need to lighten the hair beforehand by providing a combination of colour effect materials and a coating medium comprising a combination of components without the need to pre-lighten hair.

In a first aspect, the invention provides a temporary hair colour composition as defined in the accompanying claims.

In a second aspect the invention provides a method of colouring hair as defined in the accompanying claims. Suitably, the method comprises applying to the hair a temporary hair colour composition comprising an encapsulated liquid crystal and optionally an iridescent component and a coating composition to coat hair fibres and retain a natural feel while providing a vibrant colour effect, the coating composition comprising a thickening agent and a rheology modifier which comprises a glycol

The temporary hair colour composition may be used on any type of human hair including head hair, facial hair and bodily hair and is especially suitable for head hair and for the styling of beards and moustaches. The composition may also be used on any type of mammal for aesthetic or functional purposes, for example on domestic pets such as cats and dogs and on farm animals.

The thickening agent is suitably selected from a cross-linked polyelectrolyte and a hydrophobically modified alkali swellable emulsion thickening product. Suitably, the coating composition is film-forming such that the temporary hair colour composition is coatable onto hair to form a continuous film and adhere to the hair. Preferably, the film has a uniform thickness upon application to the hair so as to enable maximum reflectance of incident light on the hair enhancing colour intensity and vibrancy and feel. The thickening agent or rheology modifier or both may provide a film-forming function or an additional film-forming component may be included in the composition.

The term "temporary" as employed means a hair colour composition which has resistance to standard washing, for example in a shower and according to different hair types provided that the composition may be removed by washing i.e. it is not covalently bound to the hair. Suitably, the temporary hair colour composition is tailored to be washed out after 1 or 2 washes (referred to as 'hair makeup'), after 3 to 10, for example, 6 washes (referred to as 'temporary hair colour') or after 10 or more, for example 12, washes (referred to as 'pseudo-semi-permanent').

With known hair colour products, difficulties have been encountered in providing temporary colour effects to medium or darker coloured hair other than by pre-treating the hair to lighten its colour for example by bleaching and then applying the desired finish colour.

We have surprisingly found that the temporary hair colour composition provides a means of applying a temporary colour to darker or dark hair such that the colour or colour-change of the treated hair is readily observable without having to lighten the hair prior to application of the temporary hair colour composition.

In a further aspect, the invention provides a method of colouring hair in a colouring process wherein the hair is not lightened as a result of a lightening pre-treatment process before the colouring process, which colouring process comprises applying a temporary hair colour composition comprising a colour effect composition and a coating composition to coat hair fibres, the colour effect composition comprising an encapsulated liquid crystal and optionally an iridescent component and the coating composition comprising a thickening agent and a rheology modifier which comprises a glycol.

Advantageously, the hair colour composition of the invention provides a method of colouring hair without damaging the hair during or after use or requiring any other treatment which may cause damage to the hair, for example lightening and bleaching. The invention is particularly suitable for colouring unbleached hair, especially dark hair. The composition of the invention enables bright vibrant colour effects, for example bright green, bright blue and bright red, to be provided to unbleached dark hair.

The term "dark" is employed to indicate a range of different hair shades which conventionally prove difficult to dye effectively so as to produce an aesthetically desirable finish. Shades of hair colour may be categorised on a semi-quantitative basis across a scale of values from 1 to 12 based on the Igora Royal Natural Shades Number of Schwarzkopf. The scale provides a comparative basis for assessing the darkness and lightness of one hair shade as compared to another as set out below.

| Base colour | Black | Dark brown | Medium brown | Light brown | Dark Blonde | Medium blonde | Light blonde | Extra light blonde | Ultra blonde | Special blonde |
|---|---|---|---|---|---|---|---|---|---|---|
| Igora Shade | 1-0 | 3-0 | 4-0 | 5-0 | 6-0 | 7-0 | 8-0 | 9-0 | 10-0 | 12-0 |
| RGB Value (R-G-B) | 34-34-32 | 37-37-35 | 52-42-36 | 59-47-36 | 89-72-58 | 102-79-60 | 175-141-109 | 202-169-127 | 209-188-157 | 218-199-162 |

An "R-G-B value" may be correlated with the Igora Shade Number for each of the values 1 to 12. The R-G-B value is based on analysis of a pixelated photograph of the particular hair colour shade in which the number of red, green and blue pixels is counted based on a maximum of 250 pixels per colour. The pixelated photograph is an averaged representation of 5 representative photographs of the hair shade each of which photographs has been pixelated. The mean number of the red, green and blue pixels in each photograph is recorded and the number of red, green and blue number of pixels of the 5 photographs is averaged to provide the RGB average values for each hair shade set out in the above table. Images of hair may then be pixelated and compared with the scale in the table to determine the colour category into which the hair falls.

Advantageously, the hair colour composition of the invention may be applied to hair having any base colour and provide desirable colour effects from hair having a black or dark brown base colour to hair having an ultra-blonde or special blonde base colour.

Where hair has been treated with a semi-permanent or permanent dye, including a dark dye, the composition of the invention may still be applied as a surface, temporary coating to the hair and the composition may be tailored such that the underlying colour of the hair may be visible under certain circumstances, for example where the liquid crystal is in a clear state without the need to pre-treat the hair to lighten it.

The temporary hair colour composition suitably provides an intense and vibrant colour. Advantageously the present invention provides a liquid crystal-containing composition which may be adhered to hair and which provides excellent colour intensity without compromising the "feel" of the hair and without the need for a pre-treatment to lighten the hair. In referring to the "feel" of hair, once applied to the hair the treated hair is pleasant or not irritating for the wearer, tactile and feels natural to touch and does not form clumps or lead to other characteristics which are disadvantageous or undesirable as regards visual or tactile impact.

The temporary hair colour composition comprises an encapsulated liquid crystal and optionally an iridescent component. As desired other colour materials may be present in the composition, for example a dye.

The encapsulated liquid crystal and optionally iridescent component adhered to the surface of a hair fibre to provide a temporary colour effect whilst retaining desirable texture or "feel" provides an intense and preferably iridescent colour effect giving vibrancy and excellent aesthetic appeal. The liquid crystal may be selected and tailored to provide a static colour effect whereby the bulk colour does not change but there may be slight colour shifts or iridescence depending on temperature fluctuations. Alternatively, the encapsulated liquid crystal may be selected to change in state thereby providing a colour-change effect in the temperature range of intended use.

The liquid crystal in the present composition comprises thermotropic liquid crystals, which are responsive to a change in temperature. The liquid crystal suitably comprises a cholesteric or a chiral nematic liquid crystal. Chiral nematic liquid crystals provide bright, strong colour effects with high reflectance, suitable for premium products and are especially preferred. Cholesteric-type liquid crystals typically provide colour effects which are less intense than chiral nematic liquid crystals but are of lower cost and may be suited to larger volume markets. A mixture of chiral nematic and cholesteric liquid crystals may be employed to optimise product performance and cost. In a preferred embodiment, the liquid crystal suitably comprises only chiral nematic liquid crystals.

The liquid crystal is suitably encapsulated using known techniques and materials, for example cross-linked gum Arabic and gelatin, at known levels. In one embodiment, the liquid crystal may be encapsulated using materials which are not animal-derived thereby enabling use of the hair composition by people for whom ethical or religious reasons or personal preference may preclude use of products containing animal-derived material. The encapsulate may comprise a material which is capable of forming a gel or foam. In one aspect, the liquid crystal may be encapsulated using a plant-derived material, preferably a polysaccharide, for example chitin, alginate, carrageenan, pectin, hyaluronate. Suitably, the encapsulate wall material comprises a polymer or a combination of polymers. The wall material preferably comprises from 1 to 30%, more preferably 5 to 25%, for example 8% or 20% by weight of wall material based on the weight of the encapsulated liquid crystal.

Any known encapsulated liquid crystals may be employed including cholesterol-like chiral organics including cholesteryl carbonate esters, and chiral nematic liquid crystals. Suitable encapsulated liquid crystals are available from a range of sources including LCR Hallcrest.

The encapsulated liquid crystal is suitably incorporated into the composition as an aqueous slurry comprising from 10 to 90%, preferably 15 to 80% and especially 20 to 70% of the encapsulated liquid crystal in water, for example a 40% or 50% slurry of the encapsulated liquid crystal in water.

The encapsulated liquid crystal may be present in the temporary hair colour composition at any desired level to provide the desired colour effect upon application to the hair. Suitably, the encapsulated liquid crystal is present at a level of 0.5 to 70%, 10 to 60% and desirably 25 to 50% by weight of the temporary hair colour composition. Where a lower cost product is required, for example for a larger or mass-market product, the encapsulated liquid crystal is suitably present at a level of 20 to 40%. Where the hair has a lighter shade base colour, for example a blonde shade base colour, the encapsulated liquid crystal may be present at a higher level, preferably 40 to 60% and more preferably 50 to 60% by weight of the composition.

In one aspect, the colour composition is activated to provide colour change at a temperature greater than an ambient indoor temperature preferably greater than 20°, more preferably greater than 22°C, especially greater than 25°C and suitably at or below a temperature corresponding to a temperature encountered in direct sunlight in the geographical region in which the product is marketed, preferably lower than 35°C, more preferably lower than 33°C, for example at 28°C and 32°C. Suitably the composition is transparent at a temperature lower than the activation temperature such that when indoors or at an ambient temperature, the hair appears natural and shows the underlying base colour and as the temperature increases, for example on exposure to direct sunlight, the composition is activated to present the desired colour preferably in a manner which is vivid and impactful to the observer. Suitably, upon changing colour, the composition remains coloured while the temperature is up to 3°C and desirably up to 5°C above the activation temperature.

Advantageously, the composition provides a clear contrast which is readily observable to the human eye between the natural appearance of the inactivated "indoor" appearance and the "activated" appearance in direct sunlight.

The colour effect composition may optionally also comprise an unencapsulated liquid crystal.

Unencapsulated liquid crystals suitably provide an enhanced colour effect in combination with the encapsulated liquid crystal. The unencapsulated liquid crystals may be included in the composition together with or separately from the encapsulated liquid crystal.

An iridescent component may be present as an optional component of the temporary hair colour composition. Any suitable iridescent component or pigment may be employed and is preferably selected from mica and nacre. The presence of an iridescent component in the temporary hair colour composition suitably imparts or enhances an iridescent or pearlescent effect of the liquid crystal to provide a shimmering or glittering colour effect. Hitherto, this effect has not been possible to achieve with a temporary hair colour adhered to the surface of the hair whilst retaining good "feel" properties. Suitably, a multichromic effect, for example a trichromic effect, in which several colours may appear together due to reflection from the liquid crystal and the iridescent component and the angle of view of an observer may be achieved by appropriate selection of the iridescent component and liquid crystal.

Suitably, the iridescent component has a particle size from 1 to 200 microns. A particle of iridescent component being of a size at the lower end of this range desirably provides a "shimmering" effect, whereas a particle size towards the higher end of the range enables individual particles to be more readily observed and provides a "glitter" effect in the hair. In one embodiment, a combination of iridescent components are employed. Preferably the iridescent component comprises a first iridescent component having a particle size towards the lower end of the range, preferably 1 to 50 microns, and a second iridescent component having a particle size to the upper end of this range, for example 50 to 200 microns.

Examples of suitable micas include micas available from Sensient Cosmetic Technologies under the trade name Covapearl^{®} including Covapearl^{®}, Covapearl^{®} Star and Covapearl^{®} Mini Star range, micas available from BASF available under the trade names Reflecks^{™} MultiDimensions^{™} and Multireflections^{™} , Timica^{®} particularly the interference range particle size range having a particle size of 9 to 37 µm, Chione^{™}, Chroma-Lite^{®}, Duochrome^{®}, Gemtone^{®} , Cloisonné^{®} and Flamenco^{®}.

The mica may be treated to offer additional colour properties or improve adherence to the hair, for example the surface of the mica may be treated with a surface treatment agent preferably selected from alkyl silane, hydrogenated lecithin, dimethicone and copolyol.

In one embodiment, compositions according to the invention include compositions comprising a static encapsulated liquid crystal at a level of 1 to 50%, for example 5% and 30% and a mica. The mica may be present at a level of at least 0.1%, preferably at least 0.5%. Suitably, the level of mica does not exceed 10%, or 5% and especially not 2%. Preferably, the mica level is 0.5 to 2% by weight of the composition.

The colour effect components of the temporary hair colour composition may further comprise a dye. The dye is suitably incorporated into the composition as a powder, preferably a water-soluble powder, or as a solution of the dye at any desired level. The dye solution may comprise 0.01 to 10% aqueous solution of the dye. The dye solution may be incorporated into temporary hair colour composition at a level dependent on the concentration of the dye solution and the desired final content of the dye in the composition.

In a preferred aspect, the temporary hair colour composition comprise a soluble powder dye, or mixture of soluble powder dyes, at a level of 0.01% to 1%, more preferably at 0.01 to 0.3%, especially 0.1% to 0.3% by weight of the composition.

Any dyes suitable for use in hair products may be employed including the following used alone or in combination N,N -Bis(2-Hydroxyethyl)-2-Nitro-p-Phenylenediamine, 4-Amino-3-Nitrophenol, 4-Hydroxypropylamino-3-Nitrophenol, Hydroxyethyl-2-nitro-p-toluidine, Acid black 1, Acid blue 9, Acid green 25, HC Blue No. 12, 3-Nitro-p-Hydroxyethylaminophenol, 2-Amino-6-Chloro-4-Nitrophenol, Acid Red 33, HC Yellow No. 2, HC ORANGE NO. 1, Acid orange 7, acid red 52, acid red 18, FD&C Red 40 (curry red)HC YELLOW NO. 13, Basic Yellow 57, HC Red No. 3, Basic Red 76, Basic Red 51, Acid Red 92, Basic Yellow 87, Basic Orange 31, BASIC VIOLET 2, HC Blue No. 16, HC Blue No. 15 Acid Violet 43, Basic Brown 17 and dyes available from Sensient^{®} Cosmetic technologies; Arianor Jade Blue (HC Blue 15), Covalumine fire red AS (CI 15850, Alumina, Triethoxycaprylylsilane), Noir W 699 (CI 20470, Sodium Sulfate), Covalumine astral blue (CI 42090, Alumina, Triethoxycaprylylsilane), Unipure white LC986 FSP (CI 77891, Perfluorooctyl Triethoxysilane, Polyperfluoromethylisopropyl Ether), Unipure red LC3071 (Cl 15850, Aluminum Hydroxide), Unipure white LC987 GCA (CI 77891, Sodium Cocoyl Glutamate, Cystine, Lauroyl Arginine), Covarine white WN9787 (CI 77891, Aqua, Glycerin, Xanthan Gum, Sodium Citrate), Covapate Uniwhite LC 9781 (Ricinus Communis (Castor) Seed Oil, CI 77891,Polyhydroxystearic Acid), Unipure white LC981 SGP (CI 77891, Sodium Glycerophosphate), Vert Covasol W7035 (Cl 19140, CI 42090), Unipure yellow LC182 ADT-C (CI 77492,Isopropyl Titanium Triisostearate, Bis-PEG-15 Dimethicone/IPDI Copolymer, PEG-2 Soyamine), Unipure black LC989 ADT-C (CI 77499,Isopropyl Titanium Triisostearate, Bis-PEG-15 Dimethicone/IPDI Copolymer, PEG-2 Soyamine), unipure black LC988 FSP (CI 77499, Perfluorooctyl Triethoxysilane, Polyperfluoromethylisopropyl Ether), phat black DC 9206 (mixture of CI61565, CI60725, basic brown 16, acid violet 43, basic red 76, CI 26100), Arianor flash deep black (Basic Blue 99,Basic Brown 16, Basic Red 76, Basic Yellow 57, Basic Brown 17, Polyquaternium-37, Hydrolyzed Yeast Protein), D&C Green No. 8 (Solvent Green 7), FD&C Red No. 4 (Food red 1), D&C Red No. 22 (Acid Red 87), FD&C Blue 2 (Indigotine), FD&C Green 3 (Fast Green FCF), FD&C Red 3 (Erythrosine), FD&C Yellow 5 (Tartrazine) and D&C Red 27 (Solvent Red 48). Further examples of suitable dyes include products from the Unipure LC range and Covarine range from Sensient ^{®} technologies.

The liquid crystal may provide a single static colour or provide a colour change, for example a thermochromic colour change between red, green and blue, in response to a change in external stimulus such as rising or falling temperature, or a combination of both static colour and colour change. The composition may be tailored to provide a desired visual appearance for particular circumstances or moods and adapted to provide a faster or slower colour change with single or multiple colour change within a bandwidth of temperatures or other external parameters. In one aspect, the composition may comprise liquid crystals which are activated at different temperatures by tailoring the temperature profile of the liquid crystal. Different strands of hair may show colour change at different times or temperatures to provide a desirable aesthetic effect.

The encapsulated liquid crystal is suitably tuned to provide no colour change or change of state under conditions of its intended use thereby providing a static colour effect. A static colour effect may be achieved by employing a broader bandwidth such that upon reaching the starting point a colour is observed and the clearing point is above the temperature which the composition is likely to encounter in use, thus the colour remains at the starting point colour or another colour within the spectrum of visible light. Addition of dyes or pigments will enable enhancement of the visualised colour such that a specific colour will be obtained over a broad temperature range. For example red, orange or yellow may be 'covered' using green dye or pigment to obtain a green colour over the whole range.

The liquid crystal may be adapted to provide a colour-change or chromic effect. The composition may provide a change from clear to a colour, for example red or successively any other colour through the colours of the visible spectrum, from a colour through to colourless or from a first colour for example red to a second colour for example green in response to an external stimulus for example a change in temperature or by changing the angle of observation in the case of iridescent materials. The bandwidth of the liquid crystal composition may be tailored according to the desired effect according to techniques known to those skilled in the field.

The colour effect observed on longer hair may vary along the length of the hair fibre due to differences in temperature with the hair closer to the body being at a slightly higher temperature than hair more distant from the body creating an "ombre" effect. Tailoring the liquid crystal composition such that it has a narrow bandwidth may enable or augment this effect. As desired, different parts of the hair, for example the root and the tips, may be coloured differently so as to provide a colour gradient.

Compositions according to the invention are especially suitable for use as a hair colour-change composition. The composition may be applied to natural hair, to artificial hair, for example hair extensions, or both. The composition may be formulated to provide multiple colour changes. The colour changes may occur in a desired sequence of particular colours by suitable selection of thermochromic liquid crystal. Suitably, the composition provides two to six colour changes, respectively referred to as dichromic, trichromic, tetrachromic, pentachromic and hexachromic, with each colour change occurring at a different temperature.

The thickening agent in the coating composition is suitably selected from a cross-linked polyelectrolyte and a hydrophobically modified alkali swellable emulsion thickening product. Cross-linked polyelectrolytes are preferred. Suitably, the coating composition is film-forming such that the temporary hair colour composition is coatable onto hair to form a continuous film and adhere to the hair. Preferably, the coating composition is transparent so as to enable maximum reflectance of incident light, improving colour vibrancy and intensity.

Suitable cross-linked polyelectrolytes comprise a dissociating group in at least some, and desirably all of the monomeric units of the polymer, capable of dissociating in a polar and especially aqueous environment. Preferably the monomeric units comprise a group selected from -COOH, -SO₃H, -PO₃H₂ and -NH₂. Suitably, the cross-linked polyelectrolyte is able to form a swellable structure which swells on contact with the polar environment and desirably forms regions of micro-gels. Suitably the microgels have a size of 0.1 to 50,preferably 0.5 to 20, more preferably 1 to 10 microns, for example 3 to 4 microns so as to form around the encapsulated liquid crystal and aid its suspension within the composition which is believed to improve the uniformity of deposition of the composition on the hair.

The cross-linked polyelectrolyte may comprise a natural thickener and/or preferably a synthetic thickener. Examples of natural thickening agents include carboxymethyl cellulose, alginate, chitosan, pectin. Examples of suitable synthetic polyelectrolytes include polymers and copolymers comprising polyacrylic acid, polymethacrylic acid, polystyrene sulfonate and derivatives of these polymers and their salts. Examples of natural and synthetic polyelectrolytes are disclosed in "A comprehensive review on polyelectrolyte complexes", Meka et al, Drug Discovery Today, Vol 22, No 11, November 2017.

Reference herein to acrylic or acrylate includes methacrylic and methacrylate *mutatis mutandis* unless otherwise stated.

Suitably, the cross-linked polyelectrolyte comprises a polymer which has been modified so as to comprise a hydrophilic region and a hydrophobic region. The thickening agent preferably is soluble in water at 25°C at a level of at least 1% by weight, preferably at least 1.2% by weight and especially at least 1.5% by weight provided that the temporary hair composition enables a smooth application to the hair without compromising the feel of the hair. More preferably, the thickening agent is soluble in water at 25°C at a level of 2% by weight.

Preferably, the cross-linked polyelectrolyte comprises a polyacrylate polymer, cross-polymers and copolymers of (poly)acrylates and polyacrylic acids, for example polyacrylate crosspolymer-6-2-methyl propan-2-ol, alkyl acrylates, urethane acrylates, hydrophilically modified starch, for example hydroxypropyl starch modified with a COOH, -SO₃H, -PO₃H₂ and -NH₂ group, polyvinyl pyrollidone/vinyl acetate copolymers, polyquaternium compounds.

Examples of suitable cross-linked polyelectrolytes include SEPIMAX ZEN available from Seppic, STRUCTURE XL and DYNAMX H2O available from AkzoNobel, CARBOPOL and FIXATE FREESTYLE range of polymers available from Lubrizol including CARBOPOL^{®} Ultrez 30, CARBOPOL ^{®} Ultrez 21, COVACRYL range of polymers available from Sensient including COVACRYL P12, COVACRYL E14 WP, COVACRYL A15 WP, LUVISKOL range of polymers available from BASF including LUVISKOL VA 64, LUVISKOL VA 37-E, TRIquat range of polymers available from Tri-K including TRIQ!UAT FORCE, CELQUAT range of polymers available from AkzoNobel including CELQUAT L-200.

Where a hydrophobically modified alkali soluble polymer is employed as a thickening agent, the hydrophobically modified alkali soluble polymer is preferably an emulsion (HASE).

The hydrophobically modified alkali soluble polymer suitably comprises a polymer or a copolymer having one or more monomeric units derived from one or more of acrylic acid, methacrylic acid, hydroxyethyl methacrylic acid, alkyl preferably C₁ to C₄, methacrylic acid, alkyl preferably C₁ to C₄, acrylic acid, alkyl preferably C₁ to C₄, amino acrylic acids, acrylamides, esters of any such acrylic or methacrylic monomers and ethers of any such acrylic or methacrylic monomers, acrylated dimethiconol. Suitably the polymer is hydrophobically modified by a modified polyalkylene glycol having a mid to longer chain alcohol for example modified polyethylene glycol, modified polypropylene glycol and modified polybutylene glycol.

Preferably, the hydrophobically modified alkali soluble polymer comprises a polymer or copolymer derived from one or more of acrylic acid methacrylic acid, hydroxyethyl methacrylic acid, alkyl preferably C₁ to C₄, methacrylic acid, alkyl preferably C₁ to C₄, acrylic acid, alkyl preferably C₁ to C₄, amino acrylic acids, acrylamides, esters of any such acrylic or methacrylic monomers and ethers of any such acrylic or methacrylic monomers wherein hydrophobic modification is provided by a polyalkylene glycol having one end derivatised with a hydrophobic alcohol. The modified polyalkylene glycol preferably comprises polyethylene glycol, polypropylene glycol or polybutylene glycol, having one end modified by etherification with a hydrophobic alcohol preferably having from 6 to 40 carbon atoms, especially 10 to 30, for example behenyl alcohol.

In a preferred embodiment the hydrophobically modified alkali soluble polymer comprises a copolymer of an acrylate and a methacrylate derivatised with a modified alcohol end-capped polyalkylene glycol as described in the preceding paragraph. An example of an especially preferred polymer is ACULYN 28 ^{®} and ACULYN 33 ^{®}, available from Dow.

Suitably, the hydrophobically modified alkali soluble polymer is neutralised using a pH modifier to a pH of 6 to 8, preferably 6.5 to 7.5. A pH modifier may be required depending on the polymer employed in order to ensure the polymer does not adversely disrupt the encapsulation wall of the colour-change composition and to facilitate optimal thickening of the formulation. pH modifiers known for use in personal care products may be employed in the present composition. Examples of alkaline the pH modifiers include comprises an aminated alcohol, for example aminomethyl propanol and triethanolamine which may also provide a foam stabilizer or surfactant function. Examples of acidic pH modifiers include citric acid and sodium gluconate, which may also provide a humectant function. Examples of suitable bases include AMP Ultra 2000 ^{®} available from Angus Chemical Co, and sodium hydroxide. AMP Ultra 2000 is especially preferred as providing excellent viscosity and stability.

Suitably, the thickening agent is present at a level of 0.1 to 30%, preferably 0.5 to 15%, especially 0.75 to 10% of the temporary hair colour composition.

The rheology modifier suitably comprises a glycol selected from polyalkylene glycol, preferably polyethylene glycol, polypropylene glycol, or polybutylene glycol, and a C₂ to C₄ alkylene glycol. The polyalkylene glycol preferably comprises units of ethylene glycol, propylene glycol and/or butylene glycol. The polyalkyene glycol may comprise two or more different glycol units and comprises a block copolymer or a random copolymer but preferably comprises one glycol unit, especially ethylene glycol. The polyalkylene glycol suitably comprises from 5 to 100 alkylene glycol units, preferably from 8 to 75 alkylene glycol units, more preferably from 15 to 50 alkylene glycol units, especially from 20 to 40 alkylene glycol units. The polyalkylene glycol may comprise a blend of two or more different polyalkylene glycols.

Examples of preferred glycols include PEG 32 and propylene glycol. The rheology modifier, if present, is suitably present at a level of 0.1 to 20%, preferably 1 to 10% for example 7% by weight of the binder composition.

Advantageously, the glycol aids dispersion or solubilisation of the encapsulated liquid crystal in the composition and does not compromise the integrity of the encapsulate walls. Suitably the glycol also acts as a humectant to retain moisture, aiding improved "feel" of the hair fibres.

The coating composition suitably is film-forming so as to provide an even deposition of the liquid crystal on the hair. Examples of suitable film-forming components include the COVACRYL ^{™} range of products available from Sensient Cosmetic Technologies, including COVACRYL P12, COVACRYL MT10, COVACRYL MS11 WP, COVACRYL E14 WP; DYNAMX H2O^{™}, STRUCTURE 2001^{™}, CELQUAT L-200^{™}, RESYN 28-2930^{™}, BALANCE 47 ^{™} and BALANCE RCFg^{™} (available from Akzo Nobel), PVP/VA Copolymer 60/40 W NP, the FIXATE ^{™} range of products including FREESTYLE^{™}, SUPERHOLD^{™} G-100, available from Surfachem; GLYCOFILM 1.5P, TRIQUAT FORCE (Tri-K Industries) and LUVISKOL VA ^{™} range of products (BASF).

The thickening agent and/or rheology modifier may also provide a film-forming function or this function may be provided by an additional component of the coating composition. The film-forming component, if present as an additional component in the composition is suitably present at a level of 0.5 to 10%, preferably 0.5 to 7%, especially 1 to 5% by weight of the temporary hair colour composition.

In a preferred embodiment, the coating composition comprises a thickening agent selected from one or more of:
i) an acrylate copolymer
ii) an acrylate crosspolymer-6-2-methyl propan-2-ol;
iii) an acrylate/C₁₀₋₃₀ crosspolymer;
iv) a cross-linked homopolymer of polyacrylic acid;
v) an acrylate and urethane copolymer;
vi) a polyvinylpyrollidone and vinyl acetate copolymer;
vii) a copolymer of acrylate and steareth-20 methacrylate.
and a rheology modifier comprising polyethylene glycol.

In a further preferred embodiment, the thickening agent is selected from SEPIMAX ZEN and ACULYN 28 and optionally further comprises a film-forming component, preferably, at a level of 1 to 3% by weight.

In a preferred embodiment, the thickening agent may also provide a fixative effect by aiding adhesion of the liquid crystal to the hair without compromising feel or a separate fixative component may be employed in combination with the thickening agent. Suitably the fixative is present at a level of 0.1 to 5%, preferably from 1 to 3.5%.

Suitably, the coating composition comprises a conditioning agent. Suitable conditioning agents include moisturisers and/or humectants, examples of which include sugar alcohols, glycerin and glycols. In some embodiments, the conditioning agent is suitably selected from one or more of Keravis PE^{™} (Croda), Polyderm PPI-CO^{™} (Aston Chemicals), PEG-8, polyquarternium compounds, suitably in the range polyquaternium-4 to polyquarternium-39 (Aqua (and) Hydrolyzed Vegetable Protein PG-Propyl Silanetriol), D-Panthenol, Glycofilm 1.5P, BELSIL^{™} PDM 20 (Wacker-Chemie), NATPURE FEEL-M ECO^{™} (Sensient Cosmetic Technologies).

We have found that an anionic polysaccharide is advantageous in enhancing colour vibrancy and adhesion of the hair composition to the hair. An example of a preferred anionic polysaccharide is the GLYCOFILM range of products available from Aston Chemicals, for example GLYCOFILM 1.5P Suitably, anionic polysaccharide is present at a level of 0.1-5% by weight, preferably 0.5 to 3%, for example 1% or 1.5%.

The temporary hair composition is suitably substantially free of silicones and silicone-containing components as such components may adversely affect the encapsulated liquid crystal and disrupt the liquid crystal structure.

Whilst known pigments or dyes may be adhered to substrates using a composition containing a solvent, for example to impart flexibility and durability, organic solvents may damage the encapsulation material of an encapsulated liquid crystal.

The temporary hair colour composition of the invention is suitably an aqueous composition and suitably comprises water as a solvent for the composition. Preferably the aqueous solvent is selected from water and a mixture of water and a polar organic solvent. Polar organic solvents may be present but are suitably present at a level of less than 20%, preferably less than 10% and desirably less than 5% by weight of the composition of the invention. Any organic solvents or other organic components of the composition are suitably selected so as not to disrupt the walls of the encapsulate.

Preferably, the solvent is present in the temporary hair colour composition at a level of 30 to 95%, more preferably 50 to 90%, especially 60 to 80& for example 70% by weight of the composition.

Other optional components may be included in the temporary hair colour composition as desired including any one or more of a UV stabiliser, a chelating agent, for example disodium pyrophosphate, a preservative for example Germaben II, conditioning oil, an emulsion a conditioning colour agent and a dye.

In one embodiment, the composition may further comprise a UV stabiliser. Encapsulated liquid crystals may be prone to degradation due to the impact of UV radiation. A UV stabiliser provides improved resistance of the liquid crystal encapsulate to such degradation. Any known cosmetically approved UV stabilisers that are compatible with the encapsulate may be employed. Preferred UV stabilisers include Benzophenone-4, Avobenzone, Homosalate, Octocrylene, Benzophenone-5, Ethylhexyl Dimethyl PABA, Titanium oxide, Zinc oxide, Phenylbenzimidazole sulfonic acid, PEG-25 PABA, disodium phenyl dibenzimidazole tetrasulfonate.

The composition may also comprise a preservative such as a natural or synthetic antimicrobial. The preservative is suitably present at a level which provides the required preservative effect having regard to the product requirements, storage and market requirements and is preferably of 0.1 to 2% by weight of the composition for example 0.5%. Examples of natural antimicrobials include, carylyl glycol, tea tree oil, potassium sorbate and sorbic acid. Examples of synthetic antimicrobials include phenoxyethanol, benzylalcohol and gluconolactone. Examples of other antimicrobials include benzoic acid, sodium benzoate, sorbic acid, methylparaben and propylparaben.

In a preferred embodiment, the composition of the invention comprises:
a thickening agent selected from a cross-linked polyelectrolyte, and a hydrophobically modified alkali soluble emulsion (HASE) at a level of 0.1 to 30%, preferably 0.5 to 15%, especially 0.5 to 5% of the temporary hair colour composition;
a polyalkylene glycol at a level of 1 to 10%, preferably 1 to 7%, especially 2 to 5% of the temporary hair colour composition;
an encapsulated liquid crystal slurry, suitably comprising 30 to 60% solids in the slurry, for example 40% and 50%, at a level of 1 to 60%, preferably 1 to 50%, especially 5 to 50% or 5 to 40% of the temporary hair colour composition;
optionally an iridescent component at a level of up to 10%, especially 0.1 to 3% by weight of the temporary hair colour composition;
optionally, where the thickening agent comprises a HASE requiring neutralisation, a neutralising agent at a level sufficient to neutralise the HASE to a pH of 6 to 8, preferably 6.5 to 7.5, especially 7 to 7.5; and
water to 100%, preferably comprising at least 50 % and more preferably at least 65% of the composition.

The preferred compositions in further preferred embodiments may comprise one or more of the following optional components a preservative, a conditioning oil, an emulsion a conditioning colour agent and a dye.

The temporary hair colour composition preferably has a pH in the range 3 to 8, more preferably 3 to 7.5, particularly 5 to 7.5 and especially 6.5 to 7.5.

The temporary hair colour composition may be produced as a complete formulation which is ready-to-use by the user. Alternatively, the composition may be provided as several formulations which are to be mixed at the point-of-use comprising a coating composition comprising the thickening agent and a glycol and a colour composition comprising an encapsulated liquid crystal. The iridescent component and other optional components may be contained in the coating composition or the colour composition as desired.

The composition according to the invention may be in any suitable form, for example a gel or a cream, preferably a gel.

In the method of the invention, the temporary hair colour composition may be applied to the hair in a process comprising a step of heating the hair. Preferably, the composition is applied to the hair without the application of external heat. In a preferred embodiment, the temporary hair colour composition is applied to the hair at a temperature below 40°C and more preferably at ambient temperature.

The hair colour composition may be applied to the hair using fingers or a conventional applicator, for example a brush, or by any other known hair painting technique.

In a further aspect, the invention provides for the use of a temporary hair colour composition according to the invention to cosmetically treat hair. Suitably, the cosmetic treatment comprises at least one of applying a colouring to the hair, applying a colour-change effect to the hair or applying an iridescent effect to the hair.

The invention is illustrated by the following non-limiting examples. All percentages and parts are parts by weight and all measurements made are at 25° C, unless otherwise stated

### Example 1

A composition according to the invention was prepared having the components listed in Table 1. The components for each phase were mixed and the phases mixed by mixing phase A and B, then phase C was added. Phases E and F can then be added separately in either order or together. Phase C was added after mixing phases A and B but may be added subsequently at any time if desired. Phase D was added at the end of the formulation for pH adjustment.

**Table 1**

| Phase | Raw material | Usage Level (%) |
|---|---|---|
| A | Water | 38% |
| | SEPPIMAX ZEN (thickening agent) | 1% |
| | Glycerol | 0.5% |
| | Propylene glycol (rheology modifier) | 2.5% |
| | PEG-32 (rheology modifier) | 4% |
| | Sodium benzoate | 0.4% |
| | | |
| B | Apricot Kernel oil | 0.5% |
| | PEG-40 Castor oil | 1% |
| | Water | 1% |
| | | |
| C | Mica | 5% |
| | Encapsulated liquid crystal (40% slurry) | 25% |
| | | |
| D | Citric Acid | 0.2% |
| | | |
| E | KERAVIS PE | 0.5% |
| F | Dye (0.5% solution) | 20% |
| | | |
| | | 100% |

PEG-32 enhances the vibrancy and brightness of the colour effect by enhancing colour refraction. Glycerol and propylene glycol enhance the feel of the hair when treated with the composition. Sodium benzoate is a preservative and the pH is suitably adjusted to pH 2.5-4.0 using citric acid to optimise its functionality. Apricot kernel oil acts as a conditioning oil. EMULGIN L, an emulsifier, is available from BASF. KERAVIS PE, available from Croda, acts as a conditioning and colouring agent.

The product provided a static colour effect with an iridescent/chrome appearance and was in the form of a uniform translucent/turbid gel.

### Example 2

A composition according to the invention was prepared having the components listed in Table 2. The components for each phase were mixed and the phases mixed by mixing phase A and B, then phase D was added. Phase C was added after mixing phases A and B but may be added before, together with or after Phase D.

**Table 2**

| Phase | Raw material | Usage Level (%) |
|---|---|---|
| A | Water | 43.7 % |
| | SEPPIMAX ZEN | 1% |
| | Glycerol | 1% |
| | Germaben II | 0.8% |
| | Propylene glycol | 1% |
| | KERAVIS PE | 1% |
| | PEG-32 | 4% |
| | | |
| B | Apricot oil | 0.5% |
| | PEG-40 hydrogenated castor oil | 1% |
| | Water | 1% |
| | | |
| C | Encapsulated liquid crystal (40% slurry) | 25% |
| | | |
| D | Dye (0.5% solution) | 20.00% |
| | | 100% |

The product has a trichromic tonal shift/static colour effect with an iridescent/chrome appearance and was in the form of a gel.

### Example 3

A composition according to the invention was prepared having the components listed in Table 3. The components for phase A were mixed, after which phase B was added to phase A until complete dispersion. Phase C can be added to phase A and B or it can be mixed into phase D and then C and D mixed into Phase A and B. Phase D is a 20% mixture of water soluble dyes/ iridescent pigments. The composition has a pH between 5-6. No adjustment of the pH using a neutralising agent was necessary.

**Table 3**

| Phase | Raw material | Usage Level (%) |
|---|---|---|
| A | Water | 9% |
| | Glycerol | 0.5% |
| | Preservative | 0.5% |
| | Propylene glycol | 3% |
| | Conditioning agent | 6% |
| | LUVISKOL Film forming polymer | 5% |
| | Glycofilm 1.5P | 1.0% |
| | | |
| B | SEPPIMAX ZEN | 1% |
| | | |
| C | Encapsulated Liquid crystal (40%) slurry | 54% |
| | | |
| D | Water | 1% |
| | Dye (0.5% solution) | 15% |
| | Micas | 4% |
| | | to 100% |

### Example 4

Compositions according to the invention were prepared having the components listed in Table 4. A range of compositions were prepared with differing Phase E components comprising water soluble dyes and optionally other aesthetic components for example iridescent pigments. The components for phase A were mixed, after which phase B was added to phase A until complete dispersion occurred. Phase C can be added to phase A and B or it can be mixed into phase D and/or E and the mixture then mixed into Phase A and B. Phase D and Phase E may be added to the composition at any time. A proportion of the water included in Phase A, for example 10 to 15\5 of the total composition, may be introduced to the composition as a component of Phase E.

**Table 4**

| Phase | Raw material | Usage Level (%) |
|---|---|---|
| A | Water | 34% |
| | Disodium pyrophosphate | 0.1% |
| | Propylene glycol | 3% |
| | PVP/VA Copolymer 60/40 W NP | 3% |
| | Preservative | 0.5% |
| | | |
| B | SEPPIMAX ZEN | 1% |
| | | |
| C | Encapsulated Liquid crystal (40%) slurry | 50% |
| | | |
| D | Perfume | 0.25% |
| | | |
| E | Dyes | 0.1-1% |
| | | |
| | | to 100.00% |

The dyes may be selected according to the desired effect and also according to the base shade of the hair to which the composition is to be applied. Different dyes may be selected according to whether the base shade is dark brown, light brown, blonde or other base shades.

### Example 5

A range of compositions having the compositions of Example 1 to 4 may be prepared in which the mica is selected from the following materials, available from BASF:
- soft sparkle rose 480P, mica based Multireflections, colour variable
- twisted terracotta G390D, mica based Multireflections, colour variable
- shifting sapphire G680D, mica based Multireflections, colour variable
- glistening gold G280D, mica based Multireflections, colour variable
- Black MN4498, Timica
- Snowfall white S130D, Chione
- rouge flambe, 440X, Cloisonne
- YG gold green 822C, Duocrome
- sparkle blue 626J, Cloisonne
- goldstone G014, Gemstone colours
- tan opal G005, Gemstone colours
- moonstone G004, Gemstone colours
- violet 520C, Flamenco
- shimmering gold G230Z, Reflecks
- red MN 4506, Timica terra colours
- violet 530FR, Timica interference
- Bronze 240AB, Timica nu-antique
- sparkle copper 350J, Cloisonne sparkle colours
and Covapearl^{®}, Covapearl^{®} Star and Covapearl^{®} Mini Star range, micas available from Sensient.

### Example 6

Composition produced in Examples 1 to 4 were applied to dark hair and light hair and found to provide an excellent combination of colour intensity, vibrancy, whilst retaining excellent feel properties. When applied to dark hair, the compositions provided bright observable colours.

## Claims

1. A temporary hair colour composition comprising an encapsulated liquid crystal at a level of 10 to 60% by weight of the said composition and a coating composition comprising a thickening agent which comprises a cross-linked polyelectrolyte and/or a hydrophobically modified alkali swellable emulsion thickening product and a rheology modifier which comprises a glycol and the hair colour composition optionally further comprising an iridescent component.

2. A temporary hair colour composition according to claim 1 wherein the glycol is selected from polyalkylene glycol and a C₂ to C₄ alkylene glycol.

3. A temporary hair colour composition according to claim 2 wherein the rheology modifier comprises at least one of polyethylene glycol having from 20 to 40 glycol units and propylene glycol.

4. A temporary hair colour composition according to any one of the preceding claims wherein the thickening agent comprises a cross-linked polyelectrolyte.

5. A temporary hair colour composition according to any one of the preceding wherein the thickening agent comprises a cross-linked polyelectrolyte comprising one or more of a polyacrylate polymer, a cross-polymer and a copolymer of (poly)acrylates and polyacrylic acids, an alkyl acrylate, a urethane acrylate, a hydrophilically modified starch a polyvinyl pyrollidone/vinyl acetate copolymer and polyquaternium compound.

6. A temporary hair colour composition according to any one of the preceding claims wherein the thickening agent comprises one or more of:
i) a cross-polymer of a (poly) acrylate
ii) an acrylate copolymer
iii) an acrylate crosspolymer-6-2-methyl propan-2-ol;
iv) an acrylate/C₁₀₋₃₀ crosspolymer;
v) a cross-linked homopolymer of polyacrylic acid;
vi) an acrylate and urethane copolymer;
vii) a polyvinylpyrollidone and vinyl acetate copolymer;
viii) a copolymer of acrylate and steareth-20 methacrylate.

7. A temporary hair colour composition according to any one of the preceding claims further comprising an anionic polysaccharide.

8. A temporary hair colour composition according to any one of the preceding claims further comprising an iridescent component.

9. A temporary hair colour composition according to claim 8 wherein the iridescent component comprises:
i) an iridescent component having a particle size of 1 to 50 microns; and/or
ii) an iridescent component having a particle size of 50 to 200 microns..

10. An aqueous temporary hair colour composition according to any one of the preceding claims wherein:
i) the said thickening agent is selected from a cross-linked polyelectrolyte, and a hydrophobically modified alkali soluble emulsion (HASE) at a level of 0.5 to 15%, of the temporary hair colour composition;
ii) the said rheology modifier comprises a polyalkylene glycol at a level of 1 to 10% of the temporary hair colour composition; and the temporary hair colour composition further comprising:
iii) a film-forming component at a level of 0.5 to 10% by weight of the temporary hair colour composition.
iv) optionally an iridescent component at a level of up to 10% of the temporary hair colour composition; and
v) optionally, where the thickening agent comprises a HASE requiring neutralisation, a neutralising agent at a level sufficient to neutralise the HASE to a pH of 6 to 8.

11. A temporary hair colour composition according to any one of the preceding claims wherein the composition is capable of being washed out of the hair after from 1 to 2 washes.

12. A method of colouring hair comprising applying to the hair a temporary hair colour composition according to any one of claims 1 to 11 to coat hair fibres and retain a natural feel while providing a vibrant colour effect.

13. A method according to claim 12 wherein the hair to be coloured has not been subjected to a pre-treatment to lighten the hair before the colouring process, which colouring process comprises applying the said temporary hair colour composition to the non-lightened hair.

14. A method according to claim 12 or claim 13 wherein the hair is unbleached, dark, and/or mid-tone.

15. Use of a temporary hair colour composition according to any one of claims 1 to 11 to cosmetically treat hair.

## Patentansprüche

1. Temporäre Haarfärbezusammensetzung, umfassend einen eingekapselten Flüssigkristall in einer Menge von 10 bis 60 Gew.-% der Zusammensetzung und eine Beschichtungszusammensetzung, umfassend ein Verdickungsmittel, das einen vernetzten Polyelektrolyten und/oder eine hydrophob modifizierte alkaliquellbare Emulsion als Verdickungsprodukt umfasst, und einen Rheologiemodifikator, der ein Glykol umfasst, und wobei die Haarfärbezusammensetzung gegebenenfalls weiterhin eine irisierende Komponente umfasst.

2. Temporäre Haarfärbezusammensetzung nach Anspruch 1, wobei das Glykol aus Polyalkylenglykol und einem C₂- bis C₄-Alkylenglykol ausgewählt ist.

3. Temporäre Haarfärbezusammensetzung nach Anspruch 2, wobei der Rheologiemodifikator mindestens eines von Polyethylenglykol mit 20 bis 40 Glykoleinheiten und Propylenglykol umfasst.

4. Temporäre Haarfärbezusammensetzung nach einem der vorangehenden Ansprüche, wobei das Verdickungsmittel einen vernetzten Polyelektrolyten umfasst.

5. Temporäre Haarfärbezusammensetzung nach einem der vorangehenden Ansprüche, wobei das Verdickungsmittel einen vernetzten Polyelektrolyten, umfassend eines oder mehrere von einem Polyacrylat-Polymer, einem Kreuzpolymer und einem Copolymer von (Poly)acrylaten und Polyacrylsäuren, einem Alkylacrylat, einem Urethanacrylat, einer hydrophil modifizierten Stärke, einem Polyvinylpyrrolidon-Vinylacetat-Copolymer und einer Polyquaternium-Verbindung, umfasst.

6. Temporäre Haarfärbezusammensetzung nach einem der vorangehenden Ansprüche, wobei das Verdickungsmittel eines oder mehrere von Folgendem umfasst:
i) ein (Poly)acrylat-Kreuzpolymer;
ii) ein Acrylat-Copolymer;
iii) ein Acrylat-Kreuzpolymer 6, 2-Methylpropan-2-ol;
iv) ein Acrylat/C₁₀₋₃₀-Kreuzpolymer;
v) ein vernetztes Homopolymer von Polyacrylsäure;
vi) ein Urethan-Acrylat-Copolymer;
vii) ein Polyvinylpyrrolidon-Vinylacetat-Copolymer;
viii) ein Copolymer von Acrylat und Steareth-20-methacrylat.

7. Temporäre Haarfärbezusammensetzung nach einem der vorangehenden Ansprüche, weiterhin umfassend ein anionisches Polysaccharid.

8. Temporäre Haarfärbezusammensetzung nach einem der vorangehenden Ansprüche, weiterhin umfassend eine irisierende Komponente.

9. Temporäre Haarfärbezusammensetzung nach Anspruch 8, wobei die irisierende Komponente umfasst:
i) eine irisierende Komponente mit einer Partikelgröße von 1 bis 50 µm; und/oder
ii) eine irisierende Komponente mit einer Partikelgröße von 50 bis 200 µm.

10. Wässrige temporäre Haarfärbezusammensetzung nach einem der vorangehenden Ansprüche, wobei:
i) das Verdickungsmittel aus einem vernetzten Polyelektrolyten und einer hydrophob modifizierten alkalilöslichen Emulsion (HASE) in einer Menge von 0,5 bis 15 % der temporären Haarfärbezusammensetzung ausgewählt ist;
ii) der Rheologiemodifikator ein Polyalkylenglykol in einer Menge von 1 bis 10 % der temporären Haarfärbezusammensetzung umfasst; und wobei die temporäre Haarfärbezusammensetzung weiterhin umfasst:
iii) eine filmbildende Komponente in einer Menge von 0,5 bis 10 Gew.-% der temporären Haarfärbezusammensetzung;
iv) gegebenenfalls eine irisierende Komponente in einer Menge von bis zu 10 % der temporären Haarfärbezusammensetzung; und
v) gegebenenfalls, wo das Verdickungsmittel eine HASE umfasst, die eine Neutralisation erfordert, ein Neutralisierungsmittel in einer Menge, die ausreichend ist, um die HASE auf einen pH-Wert von 6 bis 8 zu neutralisieren.

11. Temporäre Haarfärbezusammensetzung nach einem der vorangehenden Ansprüche, wobei die Zusammensetzung nach 1 bis 2 Wäschen aus dem Haar auswaschbar ist.

12. Verfahren zum Färben von Haar, umfassend das Aufbringen einer temporären Haarfärbezusammensetzung nach einem der Ansprüche 1 bis 11 auf das Haar, um Haarfasern zu beschichten und ein natürliches Gefühl zu bewahren, während ein strahlender Farbeffekt geliefert wird.

13. Verfahren nach Anspruch 12, wobei das zu färbende Haar keiner Vorbehandlung zum Aufhellen des Haars vor dem Färbeprozess unterzogen worden ist, wobei der Färbeprozess das Aufbringen der temporären Haarfärbezusammensetzung auf das nicht aufgehellte Haar umfasst.

14. Verfahren nach Anspruch 12 oder Anspruch 13, wobei das Haar ungebleicht, dunkel und/oder in einem mittleren Farbton ist.

15. Verwendung einer temporären Haarfärbezusammensetzung nach einem der Ansprüche 1 bis 11, um Haar kosmetisch zu behandeln.

## Revendications

1. Composition de colorant capillaire temporaire comprenant un cristal liquide encapsulé à un taux de 10 à 60 % en poids de ladite composition et une composition de revêtement comprenant un agent épaississant qui comprend un polyélectrolyte réticulé et/ou un produit épaississant d'émulsion gonflable dans les alcalis modifié de manière hydrophobe et un modificateur de rhéologie qui comprend un glycol et la composition de colorant capillaire comprenant en outre optionnellement un composant iridescent.

2. Composition de colorant capillaire temporaire selon la revendication 1 dans laquelle le glycol est choisi parmi le polyalkylène glycol et un alkylène glycol en C₂ à C₄.

3. Composition de colorant capillaire temporaire selon la revendication 2 dans laquelle le modificateur de rhéologie comprend au moins l'un d'un polyéthylène glycol comportant de 20 à 40 unités glycol et du propylène glycol.

4. Composition de colorant capillaire temporaire selon l'une quelconque des revendications précédentes dans laquelle l'agent épaississant comprend un polyélectrolyte réticulé.

5. Composition de colorant capillaire temporaire selon l'une quelconque des revendications précédentes dans laquelle l'agent épaississant comprend un polyélectrolyte réticulé comprenant un ou plusieurs d'un polymère de polyacrylate, d'un polymère réticulé et d'un copolymère de (poly)acrylates et d'acides polyacryliques, un acrylate d'alkyle, un acrylate d'uréthane, un amidon modifié de manière hydrophile, un copolymère de polyvinyl pyrrolidone/acétate de vinyle et un composé de polyquaternium.

6. Composition de colorant capillaire temporaire selon l'une quelconque des revendications précédentes dans laquelle l'agent épaississant comprend un ou plusieurs de :
i) un polymère réticulé d'un (poly) acrylate
ii) un copolymère d'acrylate
iii) un polymère réticulé d'acrylate-6-2-méthyl propan-2-ol ;
iv) un polymère réticulé d'acrylate/C₁₀₋₃₀ ;
v) un homopolymère réticulé d'acide polyacrylique ;
vi) un copolymère d'acrylate et d'uréthane ;
vii) un copolymère de polyvinylpyrrolidone et d'acétate de vinyle ;
viii) un copolymère d'acrylate et de méthacrylate de stearéth-20.

7. Composition de colorant capillaire temporaire selon l'une quelconque des revendications précédentes comprenant en outre un polysaccharide anionique.

8. Composition de colorant capillaire temporaire selon l'une quelconque des revendications précédentes comprenant en outre un composant iridescent.

9. Composition de colorant capillaire temporaire selon la revendication 8 dans laquelle le composant iridescent comprend :
i) un composant iridescent ayant une taille de particule de 1 à 50 microns ; et/ou
ii) un composant iridescent ayant une taille de particule de 50 à 200 microns.

10. Composition aqueuse de colorant capillaire temporaire selon l'une quelconque des revendications précédentes dans laquelle :
i) ledit agent épaississant est choisi parmi un polyélectrolyte réticulé, et une émulsion soluble dans les alcalis modifiée de manière hydrophobe (HASE) à un taux de 0,5 à 15 % de la composition de colorant capillaire temporaire ;
ii) ledit modificateur de rhéologie comprend un polyalkylène glycol à un taux de 1 à 10 % de la composition de colorant capillaire temporaire ; et la composition de colorant capillaire temporaire comprenant en outre :
iii) un composant filmogène à un taux de 0,5 à 10 % en poids de la composition de colorant capillaire temporaire.
iv) optionnellement un composant iridescent à un taux allant jusqu'à 10 % de la composition de colorant capillaire temporaire ; et
v) optionnellement, lorsque l'agent épaississant comprend une HASE requérant une neutralisation, un agent de neutralisation à un taux suffisant pour neutraliser la HASE à un pH de 6 à 8.

11. Composition de colorant capillaire temporaire selon l'une quelconque des revendications précédentes dans laquelle la composition est capable d'être éliminée par lavage des cheveux après 1 à 2 lavages.

12. Procédé de coloration des cheveux comprenant l'application sur les cheveux d'une composition de colorant capillaire temporaire selon l'une quelconque des revendications 1 à 11 pour revêtir des fibres capillaires et conserver une sensation naturelle tout en fournissant un effet de couleur dynamique.

13. Procédé selon la revendication 12 dans lequel les cheveux devant être colorés n'ont pas été soumis à un prétraitement pour éclaircir les cheveux avant le procédé de coloration, lequel procédé de coloration comprend l'application de ladite composition de colorant capillaire temporaire sur les cheveux non éclaircis.

14. Procédé selon la revendication 12 ou la revendication 13 dans laquelle les cheveux sont non décolorés, foncés et/ou de teinte intermédiaire.

15. Utilisation d'une composition de colorant capillaire temporaire selon l'une quelconque des revendications 1 à 11 pour traiter des cheveux de manière cosmétique.
